# EUROPÄISCHE PATENTANMELDUNG

(11) **EP 1 297 802 A2**
(43) Veröffentlichungstag der Anmeldung: **02.04.2003**
(21) Anmeldenummer: 02021643.8
(22) Anmeldetag: 27.09.2002
(51) Int. Cl.: A61F 9/00

(54) **Tropfer, insbesondere Augentropfer**

(30) Priorität: 27.09.2001 DE 10147799
(71) Anmelder: Bünder Glas GmbH, 32257 Bünde (DE)
(72) Erfinder: Stohlmann, Erhard, 32257 Bünde (DE)
(74) Vertreter: Schirmer, Siegfried, Dipl.-Ing.

(57) **Zusammenfassung**

Tropfer, insbesondere Augentropfer, mit einem an der Tropferspitze angeordneten und einem Abrißkantenbereich aufweisenden Tropfereinsatz, wobei die Stirnseite des Abrißkantenbereichs bündig mit der stirnseitigen Begrenzung des Tropfereinsatzes oder gegenüber der stirnseitigen Begrenzung des Tropfereinsatzes zurückgesetzt verläuft.

## Beschreibung

Die Erfindung betrifft einen Tropfer, insbesondere einen Augentropfer, mit einem an der Tropferspitze angeordneten Abrißkantenbereich.

Mit den auf den Markt befindlichen Tropfern können nur bedingt kleine Tropfen erzeugt werden. Die Größe der Tropfen hängt von der äußeren Abrißkante an der Tropfenspitze ab. Da ein Bedarf an kleinen und exakten Tropfer-Volumen besteht, ist dazu ein kleiner und scharfkantiger Abrißkantenbereich erforderlich. Beim Applizieren wird die Spitze des Tropfers bis dicht an das Auge gebracht, wobei kein erkennbarer sicherer Abstand zwischen der Tropferspitze sowie dem Auge möglich ist. Durch das freihändige Tropfen entsteht mit der spitzen Konturgestaltung des Tropfers ein hohes Verletzungsrisiko.

Der Erfindung liegt die Aufgabe zugrunde, einen Tropfer der aufgezeigten Gattung so auszubilden, daß sehr kleine und volumengenaue Tropfen abgegeben werden können, ohne beim Applizieren das Verletzungsrisiko zu erhöhen.

Diese Aufgabe wird erfindungsgemäß durch einen Tropfer mit einem an der Tropferspitze angeordneten und einen Abrißkantenbereich aufweisenden Tropfereinsatz gelöst, wobei die Stirnseite des Abrißkantenbereichs bündig mit der stirnseitigen Begrenzung des Tropfereinsatzes oder gegenüber der stimseitigen Begrenzung des Tropfereinsatzes zurückgesetzt verläuft. Es besteht die Möglichkeit, den Abtißkantenbereich einstückig an den Tropfereinsatz anzuformen oder durch eine mittig in dem Tropfereinsatz eingesetzte Stahlkanüle zu bilden. Vorteilhafterweise besitzt die Stahlkanüle einen Durchmesser < 0,5 mm. Erfindungsgemäß ist die Stahlkanüle lagesicher angeordnet. In Ausgestaltung der Erfindung ist die Stahlkanüle in den Tropfereinsatz eingeklebt oder eingeklemmt. Zweckmäßigerweise weist der Tropfereinsatz einen sich nach unten konisch verjüngenden Spalt auf.

Es besteht die Möglichkeit, die Spitze der Kanüle durch einen Gummistopfen abzudecken und eine Abdeckkappe für den Tropfereinsatz vorzusehen. Die Abdeckkappe kann nach unten weisende Stege oder eine nach unten weisende zylindrische Wandung zur Auflagerung auf den Tropfereinsatz aufweisen. Die Höhe des Gummistopfens ist so abgestimmt, daß bei in Funktion befindlicher Abdeckkappe die Kanüle nur bis zu 1 mm nach unten gedrückt wird, ohne dabei einzustechen.

Bei einem bevorzugten Ausführungsbeispiel ist im unteren Bereich des Tropfeneinsatzes ein Innenfilter angeordnet, das in einer Fußverbreiterung des Tropfereinsatzes lagerbar ist.

Ausführungsbeispiele der Erfindung sind in der Zeichnung dargestellt und werden nachfolgend näher beschrieben. Es zeigen:
- Fig. 1: einen Querschnitt durch einen Tropfereinsatz mit eingesetzter Stahlkanüle und Gummistopfen sowie Abedeckkappe;
- Fig. 2: einen Querschnitt durch einen Tropfereinsatz mit einstückig angeformten Abrißkantenbereich und
- Fig. 3: einen Querschnitt durch einen Tropfereinsatz mit angeordnetem Filter.

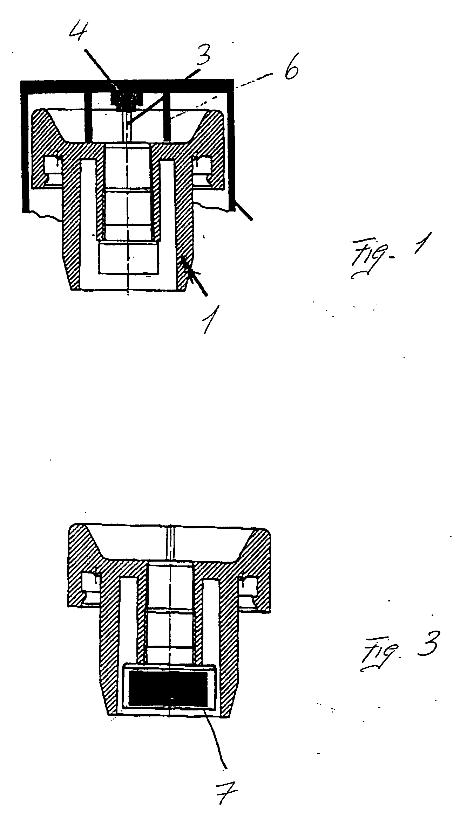

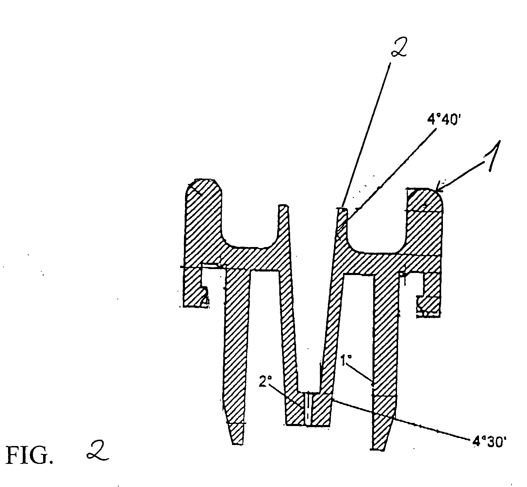

Nach Fig. 1 endet die Stahlkanüle 3 in einer Ebene mit der stirnseitigen Begrenzung des Tropfereinsatzes 1, während nach Fig. 2 der Abrißkantenbereich 2 gegenüber der Begrenzung des Tropfereinsatzes 1 zurückgesetzt verläuft. Die Spitze der Stahlkanüle 3 ist mit einem Gummistopfen 4 abgedeckt. Dem Tropfereinsatz 1 ist eine Abdeckkappe 5 zugeordnet. Diese Abdeckkappe 5 lagert mit nach unten weisenden Stegen 6 auf dem Tropfereinsatz 1 auf. Aus Fig. 3 ist der Anordnung eines Innenfilters 7 in einer Fußverbreiterung des Tropfereinsatzes 1 ersichtlich.

### Aufstellung Bezugszeichen:

- 1: Tropfereinsatz
- 2: Abrißkantenbereich
- 3: Stahlkanüle
- 4: Gummistopfen
- 5: Abdeckkappe
- 6: Stege
- 7: Innenfilter

## Patentansprüche

1. Tropfer, insbesondere Augentropfer, mit einem an der Tropferspitze angeordneten und einem Abrißkantenbereich (2) aufweisenden Tropfereinsatz (1), wobei die Stirnseite des Abrißkantenbereichs (2) bündig mit der stimseitigen Begrenzung des Tropfereinsatzes (1) oder gegenüber der stirnseitigen Begrenzung des Tropfereinsatzes (1) zurückgesetzt verläuft.

2. Tropfer nach Anspruch 1, **dadurch gekennzeichnet, daß** der Abrißkantenbereich (2) einstückig an den Tropfereinsatz (1) angeformt ist.

3. Tropfer nach Anspruch 1, **dadurch gekennzeichnet, daß** der Abrißkantenbereich (2) durch eine mittig in dem Tropfereinsatz (1) eingesetzte Stahlkanüle (3) gebildet ist.

4. Tropfer nach Anspruch 3, **dadurch gekennzeichnet, daß** die Stahlkanüle (3) einen Durchmesser < 0,5 mm aufweist.

5. Tropfer nach Anspruch 3 oder 4, **dadurch gekennzeichnet, daß** die Stahlkanüle (3) lagesicher angeordnet ist.

6. Tropfer nach einem der Ansprüche 3 bis 5, **dadurch gekennzeichnet, daß** die Stahlkanüle (3) in den Tropfereinsatz (1) eingeklebt ist.

7. Tropfer nach einem der Ansprüche 3 bis 5, **dadurch gekennzeichnet, daß** die Stahlkanüle (3) im Tropfereinsatz (1) eingeklemmt ist.

8. Tropfer nach einem der Ansprüche 3 bis 7, **dadurch gekennzeichnet, daß** der Tropfereinsatz (1) einen sich nach unten konisch verjüngenden Spalt aufweist.

9. Tropfer nach einem der Ansprüche 1 bis 8, **gekennzeichnet durch** die Anordnung eines die Spitze der Kanüle (3) abdeckenden Gummistopfens (4) und einer dem Tropfereinsatz (1) zugeordneten Abdeckkappe (5).

10. Tropfer nach Anspruch 9, **dadurch gekennzeichnet, daß** die Abdeckkappe (5) mit nach unten weisenden Stegen (6) auf dem Tropfereinsatz (1) aufliegt.

11. Tropfer nach Anspruch 9, **dadurch gekennzeichnet, daß** die Abdeckkappe (5) eine nach unten weisende innere zylindrische Wandung aufweist, die auf dem Tropfereinsatz (1) aufliegt.

12. Tropfer nach einem der Ansprüche 9 bis 11, **dadurch gekennzeichnet, daß** die Höhe des Gummistopfens (4) so abgestimmt ist, daß bei in Funktion befindlicher Abdeckkappe (5) die Kanüle (3) nur bis zu einem mm nach unten gedrückt wird, ohne dabei einzustechen.

13. Tropfer nach einem der Ansprüche 1 bis 12, **gekennzeichnet durch** die Anordnung eines Innenfilters (7).

14. Tropfer nach Anspruch 13, **dadurch gekennzeichnet, daß** das Innenfilter (7) im unteren Bereich des Tropfereinsatzes (1) gelagert ist.

15. Tropfer nach Anspruch 13 oder 14, **dadurch gekennzeichnet, daß** der Tropfereinsatz (1) zur Aufnahme des Innenfilters (7) eine Fußverbreiterung aufweist.
